# EUROPÄISCHE PATENTANMELDUNG

(11) **EP 2 441 434 A2**
(43) Veröffentlichungstag der Anmeldung: **18.04.2012**
(21) Anmeldenummer: 11183992.4
(22) Anmeldetag: 05.10.2011
(51) Int. Cl.: A61K 8/37, A61K 8/898, A61Q 5/12

(54) **Haarnachbehandlungsmittel, das besonders langanhaltenden Glanz vermittelt**

(30) Priorität: 12.10.2010 DE 102010048056
(71) Anmelder: Beiersdorf AG, 20253 Hamburg (DE)
(72) Erfinder: Saladin, Sandra, 20144 Hamburg (DE); Oelrichs, Ilka, 25436 Tornesch (DE)

(57) **Zusammenfassung**

Zubereitung zur Behandlung keratinischer Fasern enthaltend ein kationisches Polysiloxanpolymer mit Ammoniumgruppen und Polypropylenglycolbenzylethermyristat.

## Beschreibung

Die kosmetische Behandlung von Haut und Haaren ist ein wichtiger Bestandteil der menschlichen Körperpflege. Zum Standardprogramm gehören die Reinigung der Haare mit Shampoos und die darauffolgende Pflege und Regeneration mit Spülungen und Kuren. Durch diese Nachbehandlung der Haare mit Spülungen wird die Kämmbarkeit, die Geschmeidigkeit und die Frisierbarkeit der Haare verbessert sowie auch die Haare vor künftigen Schäden und Belastungen geschützt. Verbraucherinnen in Deutschland mit trockenen/strapazierten Haaren erwarten von Spülungen und Kuren vor allem eine Verbesserung der Kämmbarkeit und des Glanzes sowie eine Verringerung von Haarbruch und Spliss.

Es besteht ein Mangel an Produkten, die einen langanhaltenden Glanz erreichen.

Glanz im Sinne der vorliegenden Schrift ist der optische Eindruck des Verbrauchers beim Anschauen des Haares, den dieser als Glanz beschreibt.

Langanhaltender Glanz ist Glanz an Haaren, der bis zu 4 Stunden anhält.

Kuren und Spülungen im Sinne der vorliegenden Schrift sind Haarpflegeprodukte, die nach der Haarwäsche angewendet werden und die Eigenschaften des Haares verbessert.

Kuren sind ähnlich wie Spülungen aufgebaut, unterscheiden sich allerdings durch eine festere Konsistenz und durch einen höheren Anteil an pflegenden Inhaltsstoffen. Kuren sind für eine längere Einwirkzeit auf dem Haar vorgesehen.

Entwirrbarkeit im Sinne dieser Schrift ist die Kraft, mit der sich die nassen Haare mit dem Kamm entwirren lassen. Die Haare sind dann entwirrt, wenn der Kamm einmal komplett von oben nach unten durch die Haarsträhne geführt werden kann.

Kämmbarkeit im Sinne der vorliegenden Schrift ist die Kraft mit der sich das Haar mit dem Kamm durchkämmen lässt, nachdem die Haare entwirrt wurden. Sie wird von Probanden beurteilt. Hierbei wird eine 7er Skala verwendet bei der 7 die höchstmögliche Kämmbarkeit darstellt.

Unter Geschmeidigkeit der Haare im Sinne der vorliegenden Schrift wird das sensorische Empfinden der weichen, glatten Oberfläche der Haare verstanden.

Unter trockenen/strapazierten Haaren im Sinne der vorliegenden Schrift werden Haare von Konsumenten verstanden, die von ihnen selbst als trockene/strapazierte Haare bezeichnet werden. Ursache ist oft, dass die Haare durch Färbung, Bleichen, extreme mechanische und thermische Belastung wir Föhnen, Glätteisen und Kämmen stark beansprucht werden.

Unter Polypropylenglycolbenzylethermyristat im Sinne der vorliegenden Schrift wird das aromatische Esteröl mit der INCI-Bezeichnung PPG-3 Benzyl Ether Myristate verstanden. Dies ist bei der Gesellschaft Croda unter der Bezeichnung Crodamol STS und hat folgende Formel:

CH₃(CH₂)₁₂-(C=O)-(O-(CHCH₃)-CH₂)ₙ-OCH₂-Ph

Polypropylenglycolbenzylethermyristat ist auch in Crodazosoft SCQ (Croda) und LustreSoft (Hersteller: Croda, Inc.) enthalten, es handelt sich um Mischungen mit Quaternium-91.

Amidoamine im Sinne der vorliegenden Schrift sind Amide, die aus Fettsäuren und Diaminen gebildet werden.

In der vorliegenden Schrift angegebene Handelsnamen beziehen sich auf das jeweils zum Anmeldezeitpunkt im Markt erhältlichen Produkt.

In der vorliegenden Schrift angegebene Gehalte sind Gew.% und nicht Aktivgehalte, letztere sind wo sinnvoll angegeben.

Aus der Schrift EP 2022471 A1 war eine Conditioning Zubereitung mit Polyphenol und einem Silikonquat gewählt unter Siliconquaterniumverbindungen bekannt.

Aus der Schrift EP 2025322 A1 war eine Conditioning Zubereitung mit Polyphenol und einer Siliconquaterniumverbindung bekannt.

Aus der Schrift EP 2022478 A1 war eine Conditioning Zubereitung mit Arylsilikon und einer Siliconquaterniumverbindung bekannt.

Aus der Schrift EP 2025329 A1 war eine Conditioning Zubereitung mit Arylsilikon und einer Siliconquaterniumverbindung bekannt.

Aus der Schrift EP 1435978 A2 war eine Mischung aus Fettsäure und Estern alkoxylierter aromatischer Alkohole bekannt.

Aus der Schrift DE 102006002767 A1 war ein kosmetisches Mittel, enthaltend eine Wrkstoffkombination aus a) mindestens einer Polysiloxan Verbindung mit einer Viskosität von 0,1 cSt bis 5000 cSt und b) mindestens einem Esteröl, welches aus einer C6 bis C30 Fettsäure und einem C2 bis C30 Alkohol aufgebaut ist, bekannt.

Überraschend und für den Fachmann nicht vorhersehbar hat sich gezeigt, dass eine Zubereitung zur Behandlung keratinischer Fasern enthaltend ein kationisches Polysiloxanpolymer mit Ammoniumgruppen und Polypropylenglycolbenzylethermyristat den Nachteilen des Standes der Technik abhilft. Durch die Anwendung einer solchen Zubereitung kann ein langanhaltender Glanz, der bis zu vier Stunden anhält. Die Anwendung einer solchen Zubereitung führt auch zu einer überragenden Pflegeleistung, einer verbesserten Entwirr- und Kämmbarkeit des nassen und trockenen Haares und zu einer Verbesserung der Haarstruktur.

Besonders bevorzugt ist es, wenn das kationische Polysiloxanpolymer mit Ammoniumgruppen gewählt wird unter Silicone Quaternium-18 besonders bevorzugt Silicone Quaternium-18 von Momentive mit dem Handelsnamen Silsoft Q, Silicone Quaternium-16, besonders bevorzugt Dow Corning 5-7113, Silicone Quaternium-16/Glycidoxy Dimethicone Crosspolymer, besonders bevorzugt Dow Corning 5-7070 und/oder Silicone Quaternium-22, besonders bevorzugt ABIL T Quat 60 von Evonik. Folgende Formel 1 wurde in einem Home-in-Use Test von 50 Teilnehmerinnen in Deutschland mit trocken/strapazierten Haaren hinsichtlich Glanz besser bewertet als Formel 2.

**Tabelle 1 - Formelvergleich, Home-in-use test, Deutschland, n=50, trocken/strapazierte Haare**

| Handelsname, Anbieter, Aktivgehalt | inci | Formel 1 | Formel 2 |
|---|---|---|---|
| Solbrol M, Lanxess, 99.5% | Methylparaben | 0.25 | 0.25 |
| Propylparaben, Schütz & Co-. 99.5% | Propylparaben | 0.10 | 0.10 |
| Lanette 16, Cognis, 95% | Cetyl Alcohol | 2.20 | 3 |
| 100366 Milchsäure 90, reinst DAB, Merck, 90% | Lactic Acid | 0.80 | 0.80 |
| Nutrilan Keratin W PP, Cognis, 20% | Hydrolyzed Keratin | 0.01 | 0.01 |
| | Sodium Chloride | 0.01 | 0.01 |
| Lanette 18, Cognis , 100% | Stearyl Alcohol | 6.20 | 5.40 |
| Baumwollsaatoel, raffiniert USP 23/NF, Henry Lamotte, 100% | Gossypium Herbaceum Seed Oil | 0.01 | 0.01 |
| Gamma-Oryzanol, Biesterfeld, 98% | Oryzanol | 0.01 | 0.01 |
| Dow Corning 1501 Fluid, Dow Corning, 14% Dimethiconol | Cyclomethicone + Dimethiconol | | 2.50 |
| Xiameter PME-0245 Cyclopentasiloxane, Dow Corning, 100% | Cyclomethicone | | 1.20 |
| Dehyton AB 30, Cognis, 31% | Coco Betaine | 0.40 | 0.4250 |
| Crodamol STS-LQ-(RB), Croda, 99,95% | PPG-3 Benzyl Ether Myristate | 1.50 | 1.96 |
| Tego Amid S18, Evonik Goldschmidt, 100% | Stearamidopropyl Dimethylamine | 2.20 | 2.20 |
| Silsoft Q, Momentive Performance Materials, 20% | Silicone Quaternium-18 + Trideceth-6 + Trideceth-12 | 2 | |
| Dow Corning 5-7139, Dow Corning, 65% | Dimethicone + Cocamidopropyl Betaine + C12-15 Pareth-3 + Guar Hydroxypropyltrimonium Chloride | 1.54 | |
| TERRA-PUR Certified Spray-Dried Aloe Vera Powder, (TN001 CR), Terra Laboratories, 100% | Aloe Barbadensis Leaf Juice | 0.0010 | 0.0010 |
| Jojobaöl gepresst, Henry Lamotte, 100% | Simmondsia Chinensis Seed Oil | 0.01 | 0.01 |
| | Parfum | 0.7 | 0.7 |
| | Aqua | Ad. 100 | Ad. 100 |

Weitere relevante Leistungen der Formel hinsichtlich Pflege wie z.B. nass Kämmbarkeit, Griff der Haare und Geschmeidigkeit wurden zugunsten der Formel 1 bevorzugt.

**Tabelle 2: Ergebnisse, Home-in-use test, Deutschland, n=50, trocken/strapazierte Haare**

| n= 60 | Formel 1 | Formel 2 |
|---|---|---|
| Rücklauf n= 50 | | |
| Das Produkt macht das nasse Haar besonders leicht kämmbar | 6,0 | 5,6 |
| Das Produkt gibt dem nassen Haar einen gepflegten Griff | 5,9 | 5,4 |
| Das Produkt macht das Haar besonders weich und geschmeidig | 5,8 | 5,4 |
| Das Haar lässt sich leicht frisieren | 5,4 | 4,9 |
| Das Produkt gibt dem Haar seidigen Glanz | 5,4 | 5,0 |
| Das Produkt gefällt mir sehr gut | 5,3 | 4,8 |

Erfindungsgemäß bevorzugt ist es, wenn das kationische Polysiloxanpolymer mit Ammoniumgruppen in Konzentrationen von 0,25 bis 5 Gew.-%, bevorzugt von 0,1 bis 2,5 Gew.-% vorliegt. Weiterhin bevorzugt ist es, wenn Polypropylenglycolbenzylethermyristat in Konzentrationen von 0,5 bis 3 Gew.-%, bevorzugt 1 bis 2 Gew.-% eingesetzt wird. Besonders bevorzugt ist es, wenn das auf dem Aktivgehalt von bezogen Gewichtsverhältnis zwischen kationischem Polysiloxanpolymer mit Ammoniumgruppen und Polypropylenglycolbenzylethermyristat 1:20 bis 1:2, bevorzugt 1:10 bis 1:4 ist. Weiter bevorzugt ist es, wenn zusätzlich Amidoamine enthalten sind, insbesondere Verbindungen der Formel R¹-CONH-(CH₂)ₘ-N(R²)₂, wobei R1 gesättigte oder ungesättigte C11 bis C24-Kohlenwasserstoffketten sind, R2 C1 bis C4 Alkyl sind und m is eine ganze Zahl von 1 bis 4 darstellt. Bevorzugte Amidoamine sind Stearamidopropyldimethylamin, Stearamidopropyldiethylamin, Stearamidoethyldiethylamin, Stearamidoethyldimethylamin, Palmitamidopropyldimethylamin, Palmitamidopropyldiethyamin,
Palmitamidoethyldiethylamin, Palmitamidoethyldimethylamin,
Behenamidopropyldimethylamin, Behenamidopropyldiethylamin,
Behenamidoethyldiethylamin, Behenamidoethyldimethylamin,
Arachidamidopropyldimethylamin, Arachidamidopropyldiethylamin,
Arachidamidoethyldiethylamin, Arachidamidoethyldimethylamin,besonders bevorzugt Stearamidopropyldimethylamin, Stearamidoethyldiethylamin und deren Mischungen. Ganz besonders bevorzugt sind Stearamidopropyl Dimethylamin (Tego Amid S 18 von Evonik), Behenamidopropyl Dimethylamin (Amidet APA-22 von Kao). Dabei ist es bevorzugt, wenn Amidoamine in einer Menge von 1 bis 2,5 Gew.% eingesetzt werden. Weiter ist es bevorzugt, wenn zusätzlich Monoalkyltrimethylammoniumsalze mit einer C16 bis C24-Alkylgruppe enthalten sind, bevorzugt gewählt unter Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat, besonders bevorzugt Cetyltrimethylammonium- und Behenyltrimethylammoniumsalze ganz besonders bevorzugt in Form der Methosulfate, Chloride und Bromide, ganz außergewöhnlich besonders bevorzugt Cetyltrimethylammoniumchlorid und höchst bevorzugt Behenyltrimethylammoniumchlorid. Dabei ist es bevorzugt wenn Monoalkyltrimethylammoniumsalze in einer Menge von 0,01 bis 4 Gew.-% eingesetzt werden, bevorzugt 0,2 bis 2 Gew.-%, besonders bevorzugt 0,2 bis 1,5 Gew.-%.

Bevorzugt ist es, wenn langkettige Fettalkohole gewählt unter C14, C16, C18, C22 Alkoholen enthalten sind. Besonders bevorzugt ist es, wenn langkettige Fettalkohole in Mengen von 5 bis 9 Gew.-%, bevorzugt 6,5 bis 8,5 Gew.-%, besonders bevorzugt 7,5 bis 8,5 Gew.-% enthalten sind. Ganz besonders bevorzugt ist es, wenn zwei unterschiedliche langkettige Fettalkohole in einem Verhältnis von 2:1 bis 4:1 zueinander enthalten sind, wobei der länger kettige Fettalkohole im Überschuss vorhanden ist. Weiter ist es bevorzugt, wenn zusätzlich UV-Filter besonders bevorzugt Benzophenone-4 enthalten sind. Weiter ist es bevorzugt, wenn Oryzanol enthalten ist. Weiter ist es bevorzugt, wenn eine Komponente gewählt unter Baumwollsaatöl, Babussu Öl, Jojoba Öl, Aloe Vera, Mandelöl oder hydrolysiertes Keratin enthalten ist.

Die Erfindung umfasst auch die Verwendung einer erfindungsgemäßen Zubereitung zur Verbesserung der Dauerhaftigkeit des Glanzes.

Als weitere Ausführungsform der Erfindung können die erfindungsgemäßen Mittel weiterhin mindestens einen Emulgator enthalten. Diese können ausgewählt werden aus der Gruppe Glyceryl Stearate SE bekannt als Tegin VS von Evonik, Ceteareth-20 bekannt als Eumulgin B 2 von Cognis, Glyceryl Stearate bekannt als Tegin M von Evonik, Glyceryl Stearate Citrate bekannt als Imwitor 372 P von Sasol, Ceteareth-21 bekannt als Tego Alkanol S 21 von Evonik, Sodium Stearyl Glutamate bekannt als Eumulgin SG von Cognis und Polysorbate 60 bekannt als Tween 60 V von Croda.

Bevorzugte Menge der eingesetzten Emulgatoren beträgt 0,01 - 1,0%Gew. Kombinationen von obengenannten Emulgatoren sind auch denkbar, sowie auch folgende Zusammensetzungen: Glyceryl Isostearate/Isoceteth-20 (Tegin ISO von Evonik/Tego Alkanol IC 20 von Evonik); Ceteareth-20/Glyceryl Stearate + Ceteareth-20 + Cetearyl Alcohol + Cetyl Palmitate + Ceteareth-12 (Eumulgin B 2 von Cognis/Eumulgate SE-PF von Cognis).

Gemäß der Erfindung ist es bevorzugt, wenn ein weiteres Silikonöl vorhanden ist. Wenn als solches ein PDMS (in Form einer Emulsion verwendet werden, dann beträgt die Tröpfchengröße der emulgierten Teilchen erfindungsgemäß 0,01 µm bis 10000 µm, bevorzugt 0,01 bis 100 µm, ganz besonders bevorzugt größere Partikel von 0,3 bis 30 µm. Die Teilchengröße wird dabei nach der Methode der Lichtstreuung bestimmt. Bevorzugt sind Viskositäten der Silikonemulsionen von bis 5000 -12000 mPa.s gemessen mit dem Haake Viscotester VT 550 mit dem Meßsystem NV bei 25°C. Dimethicone kann alleine in der Emulsion sowie auch als Mischung mit einem weiteren PDMS (Polydimethylsiloxan) vorhanden sein. Beispiele sind als Handelsnamen Dow Corning 5-7139, Dow Corning 2-1352 und Dow Corning 2-1491 Emulsionen bekannt.

Dimethicone kann auch als Fluid vorhanden sein. Bekannte Handelsnamen sind Xiameter® PMX-200 Silicone Fluid Serie von Dow Corning mit kinematischen Viskositäten von 50cSt bis maximal 60000cSt. Weitere Handelsnamen für Dimethicone sind unter Wacker Belsil DM Grades, BRB Silicone Oil DM Grades, SF96 Serie von Momentive Performance Materials und DM Fluid Serien von Shin-Etsu Chemicals Co. bekannt. Ein weiteres bevorzugtes Silikonöl ist Baysilone-Oel M 100 von Momentive Performance Materials.

Dimethicone kann auch als Mischung mit einem weiteren flüchtigen oder nichtflüchtigen Silikonöl vorhanden sein.

Bevorzugt wird eine Einsatzkonzentration von <2,5 Gew.-% des PDMS zusätzlich eingesetzt.

Anstelle von Dimethicone als Silikonöl kann Amodimethicone eingesetzt werden. Amodimethicone wird durch folgende Struktur gekennzeichnet:

R-((CH₃)₂SiO)ₓ-(CH₃SiO-X-NHCH₂CH₂NH₂)_{y}--((CH₃)₂SiO)_{z}-R

R=OH oder CH₃, und X: Propyl, iso-Propyl, iso-Butyl.

Hierfür bevorzugte Amodimethicone sind als Emulsionen erhältlich unter den Handelsnamen:
Dow Corning CE-8170 AF Microemulsion, Dow Corning 929, 939, 949 und 959 Cationic Emulsion, Dow Corning 2-8177 Emulsion, Dow Corning (R) 2-8194 Microemulsion, sowie auch die Wacker Silikone Macroemulsionen Wacker-Belsil ADM 6060 und Wacker-Belsil ADM 6057E, und die Microemulsionen Wacker-Belsil ADM 8020VP und SLM 28040. Bevorzugte Amodimethicone von Momentive Performance Materials sind bekannt als SM 2125, SM 2658, SM 254, SME 253 sowie auch, Silsoft EMU 9101 N und XS65-C0032 und XS65-C0726.

Der pH Wert von einer solche Komposition wie hier beschrieben liegt zwischen 2 und 8, präferiert wird zwischen 2,5 und 6,5 und besonders präferiert ist 3,5 und 5,0.

Weiterhin können kationische Polymere wie z.B. quaternisierte Cellulose-Derivate unter dem Handelsanamen Celquat® und Polymer JR® in geringen Mengen sowie auch kationische Guar-Derivate wie unter den Handelsnamen Cosmedia® Guar und Jaguar® bekannt. Polymere Dimethyldiallylammoniumsalze und deren Copolymere mit Estern und Amiden von Acrylsäure und Methacrylsäure können ebenfalls enthalten sein. Diese sind unter den Bezeichnungen Merquat®100 (Poly(dimethyldiallylammoniumchlorid)) und Merquat®550 (Dimethyldiallylammoniumchlorid-Acrylamid-Copolymer) im Handel erhältlich. Vorteilhaft sind ebenfalls Copolymere des Vinylpyrrolidons mit quaternierten Derivaten des Dialkylaminoalkylacrylats und -Methacrylats, wie beispielsweise mit Diethylsulfat quaternierte Vinylpyrrolidon-Dimethylaminoethylme-thacrylat-Copolymere sind unter den Bezeichnungen Gafquat®734 und Gafquat®755 im Handel erhältlich. Weiterhin können Vinylpyrrolidon-Vinylimidazoliummethochlorid-Copolymere im Rahmen dieser Erfindung vorhanden sein und sind im Handel unter den Bezeichnungen Luviquat® FC 370, FC 550, FC 905 und HM 552 erhältlich. Diese sollten <1% - Gew% eingesetzt werden.

Geringe Mengen <1% amphotere Tenside haben eine strukturstabilisierende Wirkung auf die Formulierungen dieser Erfindung. Diese sollten in Konzentrationen zwischen 0,05 - 0,50 Gew.-% und besonders bevorzugt in Konzentrationen von 0,1 bis 0,30 Gew.-% vorhanden sein. Bevorzugte amphotere Tenside sind bekannt als Betaine und besonders bevorzugt ist Coco-Betaine, bekannt als Dehyton AB 30 von Cognis.

### Beispiele

Die folgenden Beispiele sollen den Gegenstand der vorliegenden Erfindung erläutern ohne ihn jedoch zu beschränken.

| Handelsname | inci | 1 | 2 | 3 | 4 | 5 |
|---|---|---|---|---|---|---|
| | Aqua | add 100 | add 100 | add 100 | add 100 | add 100 |
| 100366 Milchsäure 90, reinst DAB, Merck, 90% | Lactic Acid | 0,8 | 0,8 | 0,8 | 0,8 | 0,8 |
| Sanal P, Akzo Nobel, 100% | Sodium Chloride | 0,01 | 0,01 | 0,01 | 0,01 | 0,01 |
| Dehyton AB 30, Cognis, 31 % | Coco Betaine | 0,3 | 0,2 | 0,4 | 0,4 | 0,4 |
| Dehyquart A-CA, Cognis, 25% | Cetrimonium Chloride | | | 1,5 | | 0,8 |
| Genamin KDMP, Clariant, 83% | Behentrimonium Chloride, Isopropanol | | | | 1 | 0,6 |
| Tego Amid S 18, Evonik Goldschmidt, 100% | Stearamidopropyl Dimethylamine | 1 | 2,2 | | | |
| Silsoft Q, Momentive Performance Materials, 20% | Silicone Quaternium-18 + Trideceth-6 + Trideceth-12 | 1 | 1,5 | 2 | 2 | 2 |
| Dow Corning 959 Cationic Emulsion, Dow Corning, 50% | Amodimethicone + Trideceth-12 + Cetrimonium Chloride | | 1 | | | |
| Dow Corning 5-7139, Dow Corning, 65% | Dimethicone + Cocamidopropyl Betaine + C12-15 Pareth-3 + Guar Hydroxypropyltrimonium Chloride | 1 | | 1,54 | 1,54 | 1,54 |
| Lanette 16, Cognis, 100% | Cetyl Alcohol | 2,9 | 3,1 | 2,2 | 2,2 | 2,2 |
| Lanette 18, Cognis, 100% | Stearyl Alcohol | 5,3 | 5,6 | 6,2 | 6,2 | 6,2 |
| Uvinul MS-40, BASF, 100% | Benzophenone-4 | | 0,25 | | | |
| Crodamol STS-LQ-(RB), Croda, 99,95% | PPG-3 Benzyl Ether Myristate | 1,96 | 1,96 | 1,5 | 1,5 | 1,5 |
| Dow Corning 1501 Fluid | Cyclomethicone + Dimethiconol | | 3 | | | |
| Baysilone SF 1202 | Cyclomethicone | 1,2 | | | | |
| Methylparaben | Methylparaben | 0,25 | 0,25 | 0,25 | 0,25 | 0,25 |
| Propylparaben | Propylparaben | 0,1 | 0,1 | 0,1 | 0,1 | 0,1 |
| Neolone 950 | Methylisothiazolinone | | | | | |
| | | | | | | |
| | Parfum | 0,7 | 0,7 | 0,7 | 0,7 | 0,7 |

| Handelsname | inci | 6 | 7 | 8 | 9 | 10 |
|---|---|---|---|---|---|---|
| | Aqua | add 100 | add 100 | add 100 | add 100 | add 100 |
| 100366 Milchsäure 90, reinst DAB, Merck, 90% | Lactic Acid | 0,8 | 0,8 | 0,8 | 0,8 | 0,8 |
| Sanal P, Akzo Nobel, 100% | Sodium Chloride | 0,01 | 0,01 | 0,01 | 0,01 | |
| Magnesiumchlorid-Hexahydrat reinst DAB, Ph Eur, BP, FCC, USP, Lebensmi.qual., Merck, 100% | Magnesium Chloride | | | | | 0,02 |
| Dehyton AB 30, Cognis, 31 % | Coco Betaine | 0,4 | 0,4 | 0,4 | 0,5 | 0,4 |
| Tego Amid S 18, Evonik Goldschmidt,100% | Stearamidopropyl Dimethylamine | 2,2 | 2,5 | 2,2 | 2 | 2,2 |
| Silsoft Q, Momentive Performance Materials, 20% | Silicone Quaternium-18 + Trideceth-6 + Trideceth-12 | 2 | 1 | 2 | 2 | 2 |
| Dow Corning 959 Cationic Emulsion, Dow Corning, 50% | Amodimethicone + Trideceth-12 + Cetrimonium Chloride | | | 1,5 | | |
| Dow Corning 5-7139, Dow Corning, 65% | Dimethicone + Cocamidopropyl Betaine + C12-15 Pareth-3 + Guar Hydroxypropyltrimonium Chloride | 1,54 | 1,8 | 1 | | |
| Lanette 16, Cognis, 100% | Cetyl Alcohol | 2,2 | | 2 | 6 | 5 |
| Lanette 18, Cognis, 100% | Stearyl Alcohol | 6,2 | | 2 | 2 | 3 |
| Lanette-O, Cognis, 100% | Cetearyl Alcohol | | 7 | 3 | | |
| Crodamol STS-LQ-(RB), Croda, 99,95% | PPG-3 Benzyl Ether Myristate | 1,5 | 1,2 | 1,5 | 1 | 2 |
| Baysilone-Oel M 100 | Dimethicone | | | | 1,5 | |
| Dow Corning 1501 Fluid | Cyclomethicone + Dimethiconol | | 1 | | | 2,5 |
| Baysilone SF 1202 | Cyclomethicone | | | 1 | | |
| Methylparaben | Methylparaben | 0,25 | | 0,25 | 0,25 | 0,25 |
| Propylparaben | Propylparaben | 0,1 | | 0,1 | 0,1 | 0,1 |
| Neolone 950 | Methylisothiazolinone | | 0,05 | | | |
| | | | | | | |
| | Parfum | 0,7 | 0,7 | 0,7 | 0,7 | 0,7 |

## Patentansprüche

1. Zubereitung zur Behandlung keratinischer Fasern enthaltend ein kationisches Polysiloxanpolymer mit Ammoniumgruppen und Polypropylenglycolbenzylethermyristat.

2. Zubereitung nach Patentanspruch 1 **dadurch gekennzeichnet, dass** das kationische Polysiloxanpolymer mit Ammoniumgruppen gewählt wird unter Silicone Quaternium-18 mit dem von Momentive angebotenen Handelsnamen Silsoft Q, Dow Corning 5-7113 (Silicone Quaternium-16), Dow Corning 5-7070 (Silicone Quaternium-16/Glycidoxy Dimethicone Crosspolymer) und Silicone Quaternium-22 (als ABIL T Quat 60 von Evonik).

3. Zubereitung nach einem der vorangehenden Patentansprüche **dadurch gekennzeichnet, dass** das kationische Polysiloxanpolymer mit Ammoniumgruppen in Konzentrationen von 0,25 bis 5 Gew.-%, bevorzugt von 0,1 bis 2,5 Gew.-% vorliegt.

4. Zubereitung nach einem der vorangehenden Patentansprüche **dadurch gekennzeichnet, dass** Polypropylenglycolbenzylethermyristat in Konzentrationen von 0,5 bis 3 Gew.-%, bevorzugt 1 bis 2 Gew.-% eingesetzt wird.

5. Zubereitung nach einem der vorangehenden Patentansprüche **dadurch gekennzeichnet, dass** das auf dem Aktivgehalt von bezogen Gewichtsverhältnis zwischen kationischem Polysiloxanpolymer mit Ammoniumgruppen und Polypropylenglycolbenzylethermyristat 1:20 bis 1:2, bevorzugt 1:10 bis 1:4 ist.

6. Zubereitung nach einem der vorangehenden Patentansprüche **dadurch gekennzeichnet, dass** zusätzlich Amidoamine enthalten sind, bevorzugt Stearamidopropyl Dimethylamine (Tego Amid S 18 von Evonik), Behenamidopropyl Dimethylamine (Amidet APA-22 von Kao).

7. Zubereitung nach dem vorangehenden Patentanspruch **dadurch gekennzeichnet, dass** Amidoamine in einer Menge von 1 bis 2,5 Gew.% eingesetzt werden.

8. Zubereitung nach einem der vorangehenden Patentansprüche **dadurch gekennzeichnet, dass** zusätzlich Monoalkyltrimethylammoniumsalze mit einer C16 bis C24-Alkylgruppe enthalten sind, bevorzugt gewählt unter Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat, besonders bevorzugt Cetyltrimethylammonium- und Behenyltrimethylammoniumsalze ganz besonders bevorzugt in Form der Methosulfate, Chloride und Bromide, ganz außergewöhnlich besonders bevorzugt Cetyltrimethylammoniumchlorid und höchst bevorzugt Behenyltrimethylammoniumchlorid.

9. Zubereitung nach dem vorangehenden Patentanspruch **dadurch gekennzeichnet, dass** Monoalkyltrimethylammoniumsalze in einer Menge von 0,01 bis 4 Gew.-% eingesetzt werden, bevorzugt 0,2 bis 2 Gew.-%, besonders bevorzugt 0,2 bis 1,5 Gew.-%.

10. Verwendung einer Zubereitung nach einem der vorangehenden Patentansprüche zur Verbesserung der Dauerhaftigkeit des Glanzes.
